# EUROPEAN PATENT APPLICATION

(11) **EP 4 159 862 A1**
(43) Date of publication of application: **05.04.2023**
(21) Application number: 21812733.0
(22) Date of filing: 25.05.2021
(51) Int. Cl.: C12N 15/62, C12N 5/10, C12N 15/76, C40B 40/06

(54) **METHOD FOR PREPARING COMBINATORIAL LIBRARY OF MULTI-MODULAR BIOSYNTHETIC ENZYME GENE**

(30) Priority: 26.05.2020 JP 2020091799
(71) Applicant: Spiber Inc., Tsuruoka-shi, Yamagata 997-0052 (JP)
(72) Inventor: LIPS David, Tsuruoka-shi, Yamagata 997-0052 (JP); VERKLEIJ Gijs, Tsuruoka-shi, Yamagata 997-0052 (JP)
(74) Representative: Handley, Matthew Edward
(86) International application number: PCT/JP2021/019849
(87) International publication number: WO 2021/241593

(57) **Abstract**

The present invention relates to a method of preparing a gene cluster construct including a plurality of genes encoding a multi-modular biosynthetic enzyme, the method including (A) a step of preparing a plurality of DNA fragments which are capable of reconstructing the gene cluster construct and have structures that can be ligated to each other and (B) a step of ligating the plurality of DNA fragments prepared in the step (A) to each other by mixing the plurality of DNA fragments in a solution to obtain the gene cluster construct.

## Description

### Technical Field

The present invention relates to a method of preparing a gene cluster construct including a plurality of genes encoding a multi-modular biosynthetic enzyme and a method of preparing a combinatorial library of multi-modular biosynthetic enzyme genes from the gene cluster construct.

### Background Art

Natural compounds produced by microorganisms such as actinomycetes and filamentous fungi are known as useful substances having wide variety of structures and biological activities. In the present day, identification of gene clusters that biosynthesize the useful substances has become easy due to decoding of genome. It has also become clear that there exist many gene clusters that biosynthesize useful substances that have not been used by humans. The gene clusters that biosynthesize secondary metabolites of microorganisms have been studied, focusing on industrially important polyketide compounds and peptide compounds. For example, studies have been conducted on type I polyketide synthases (PKSs) used in the biosynthesis of macrolide compounds such as erythromycin, FK-506 (tacrolimus), rapamycin, and avermectin which are clinically applied secondary metabolites produced by actinomycetes.

Given the difficulty of producing polyketide compounds or the like by a traditional chemical method and the typically low production of polyketides in wild-type cells, there has been considerable interest in finding improved or alternative means for producing the polyketide compounds. For these reasons, heterologous production of the compounds has been attempted by introducing a gene cluster required for the biosynthesis from the original strain into other cells. The conventional method of the heterologous expression is often limited to small-sized gene clusters (< 40 kb) in practice, and many PKS gene clusters are DNAs of considerably larger sizes, ranging from several kilobases to 100 kilobases or more.

As a method of assembling DNAs, methods of ligating a plurality of DNA fragments are known, such as the Golden Gate method and the Gibson method (Non Patent Literature 1) .

The Golden Gate method is a method in which a plurality of DNA fragments are prepared, the DNA fragments containing a base sequence recognized by a type IIs restriction enzyme at one or both ends thereof, and the DNA fragments are treated with the type IIs restriction enzyme and a DNA ligase. The plurality of DNA fragments are hybridized by sticky ends generated by the type IIs restriction enzyme cleavage, and then the nicks are connected by the DNA ligase, by which a DNA fragment having a desired base sequence can be produced. The DNA fragment having a desired base sequence can be produced by designing the type and position of the base sequence recognized by the type IIs restriction enzyme, so that the plurality of DNA fragments cleaved by the type IIs restriction enzyme are ligated in such a way that there are no recognition sites for restriction enzymes. A method of using the Golden Gate method to introduce a DNA fragment having a desired base sequence into a cloning vector and the like has been reported (Patent Literature 1 and Non Patent Literature 1).

The Gibson method is a method in which a plurality of DNA fragments are prepared, the DNA fragments being designed in a manner that the ligating regions of the respective end portions in the adjacent DNA fragments to be ligated together overlap by about 15 to 80 base pairs (bp) (so that the ligating regions are the same base sequences), and the DNA fragments are treated with a 5' exonuclease, a DNA polymerase, and a DNA ligase. Due to the 5' exonuclease, single-stranded DNAs are partially formed at the ends of the DNA fragments. The single-stranded DNAs thus formed are hybridized by the overlapping base sequence portions. Then, the DNA polymerase fills the gap, and the nick is connected by the DNA ligase, whereby a DNA fragment having a desired base sequence can be produced.

Since the Gibson method does not require that the DNA fragments to be ligated together contain a base sequence recognized by a restriction enzyme or the like, there are no restrictions on the base sequences, and the method is suitable for constructing long DNA fragments (Non Patent Literature 2, Patent Literature 2, and Patent Literature 3).

### Citation List

### Patent Literature

Patent Literature 1: US 2010/0,291,633 A
Patent Literature 2: US 7,723,077
Patent Literature 3: JP 2011-512140 A

### Non Patent Literature

Non Patent Literature 1: PLoS One, 2009, 4(5), e5553
Non Patent Literature 2: Kagaku To Seibutsu, 2016, Vol. 54, No. 10, pp. 740 to 746

### Summary of Invention

### Technical Problem

The current situation is that even if a new substance is produced by a hybrid PKS module, the yield is often greatly reduced, and an efficient modified method has not been established. The cause is complex, and one possible explanation is that it is the elongation substrate that builds most of the polyketide backbone, the elongation reaction is difficult to be artificially modified, and the ketide provided by the upstream module cannot be extended.

Another possible explanation is that, since the transcriptional regulatory system of any host in the heterologous production is different from that of actinomycetes or filamentous fungi, it is necessary to replace the promoters of all genes required for the biosynthesis in order to obtain sufficient expression level. For example, by adding or replacing with strong promoters known to function in a heterologous host, it was possible to increase the productivity of polyketides in the heterologous host (Non Patent Literature 2 and Non Patent Literature 3). However, since a biosynthetic reaction of a secondary metabolite often occurs through multiple steps, it is necessary to coexpress a plurality of genes. During the coexpression, the optimal level of production cannot be achieved by expressing each gene as strongly as possible (Non Patent Literature 4), and coordinated expression is required. Considering the difficulty of predicting the ideal balance between gene expression, the most effective method of creating a biosynthetic gene cluster in a heterologous host with high productivity is creating a gene cluster library with a variety of promoter strengths and ribosome binding sites (RBSs) for regulating transcription and translation associated with the regulation of synthesis. It is considered that the combination that leads to the ideal balance between protein expression can be found by regulating the relative abundance of individual enzymes at both transcriptional and translational levels over a wide range.

Therefore, in order to simultaneously examine many combinations of PKS modules and expression parameters, a combinatorial library technique of selecting one of multiple options for each module and/or expression parameter and linking each to construct a variety of PKS gene clusters is desirable from the viewpoint of efficiency.

### Solution to Problem

The present invention provides the following inventions [1] to [11].
[1] A method of preparing a gene cluster construct including a plurality of genes encoding a multi-modular biosynthetic enzyme, the method including
   (A) a step of preparing a plurality of DNA fragments which are capable of reconstructing the gene cluster construct and have structures that can be ligated to each other and
   (B) a step of ligating the plurality of DNA fragments prepared in the step (A) to each other by mixing the plurality of DNA fragments in a solution to obtain the gene cluster construct.
[2] The method according to [1], in which the DNA fragments are each contained in plasmids, and the method further includes a step of preparing the DNA fragments of the step (A) from the plasmids.
[3] A method of preparing a gene cluster construct including a plurality of genes encoding a multi-modular biosynthetic enzyme, the method including the following steps:
   (P) a step of preparing a plurality of types of plasmids containing a plurality of DNA fragments having structures that can be ligated to each other,
   (A1) a step of preparing a mixture solution of the plurality of types of DNA fragments by treating the plurality of types of plasmids with restriction enzymes suitable to each of the plasmids, and
   (B1) a step of reaccumulating the DNA fragments using the mixture solution of the DNA fragments obtained in the step (A1) to obtain the gene cluster construct.
[4] The method according to [3], in which, in the step (P), the plurality of types of plasmids containing the plurality of DNA fragments having the structures that can be ligated to each other are prepared by using Bacillus subtilis.
[5] The method according to any one of [1] to [4], in which the multi-modular biosynthetic enzyme is a modular polyketide synthase (PKS).
[6] The method according to [3], in which the restriction enzymes are Type II restriction enzymes.
[7] A preparation method for a combinatorial library of plasmids including transforming the gene cluster construct prepared by the method according to any one of [1] to [6] into Bacillus subtilis and recovering plasmid DNAs from the Bacillus subtilis.
[8] The method according to [3], in which the step (A1) and the step (B1) are performed by selecting a plurality of types of the plasmids obtained by the preparation method according to [7] and reusing the selected plasmids as the plasmids in the step (A1).
[9] A combinatorial library of plasmids obtained by the preparation method according to [7].
[10] A method of producing a multi-modular biosynthetic enzyme including transforming the plasmids according to [9] into host cells.
[11] The method according to [10], in which the host cells are microorganisms belonging to the genus Streptomyces.

### Advantageous Effects of Invention

According to the present invention, the entire combinatorial library of a multi-modular biosynthetic enzyme, for example, PKS gene cluster library, can be assembled from a gene cluster construct synthesized by one-pot reaction. As a result, optimization of expression yield of a gene cluster and generation of chemical diversity for improving physiological activities can be expected.

### Brief Description of Drawings

Fig. 1 is a schematic diagram illustrating the procedures in Examples 1 and 2.
Fig. 2 is a photograph showing an example of restriction digestion patterns of plasmids obtained in Examples 1 and 2.
Fig. 3 is a vector map of OGAB Vector 1.0.
Fig. 4 is a vector map of OGAB Vector 2.0.
Fig. 5 is a vector map of OGAB Vector 2.1.
Fig. 6 is a vector map of OGAB Vector 2.2.

### Description of Embodiments

Hereinafter, embodiments of the invention will be described in detail. However, the invention is not limited to the following embodiments.

A method of preparing a gene cluster construct including a plurality of genes encoding a multi-modular biosynthetic enzyme according to an embodiments includes (A) a step of preparing a plurality of DNA fragments which are capable of reconstructing the gene cluster construct and have structures that can be ligated to each other and (B) a step of ligating the plurality of DNA fragments prepared in the step (A) to each other by mixing the plurality of DNA fragments in a solution to obtain the gene cluster construct.

A method of preparing a gene cluster construct including a plurality of genes encoding a multi-modular biosynthetic enzyme according to another embodiment includes the following steps: (P) a step of preparing a plurality of types of plasmids containing a plurality of DNA fragments having structures that can be ligated to each other, (A1) a step of preparing a mixture solution of the plurality of types of DNA fragments by treating the plurality of types of plasmids with restriction enzymes suitable to each of the plasmids, and (B1) a step of reaccumulating the DNA fragments using the mixture solution of the DNA fragments obtained in the step (A1) to obtain the gene cluster construct.

In the present specification, examples of the multi-modular biosynthetic enzyme include a type I polyketide synthase (may be simply referred to as a "PKS" or a "type I PKS") and a nonribosomal peptide synthetase.

By combining the gene cluster construct or the plasmid obtained in the present invention with a specific host, a useful substance biosynthesized by a multi-modular biosynthetic enzyme (for example, a polyketide or a nonribosomal peptide) can be produced with high efficiency. In one preferred embodiment of the present invention, genetically engineered host cells can be used, in which a naturally occurring gene that encodes a multi-modular biosynthetic enzyme (for example, a type I PKS gene or a nonribosomal peptide synthetase) is substantially deleted. These host cells can be transformed with plasmids containing various genes encoding multi-modular biosynthetic enzymes (for example, type I PKS genes or nonribosomal peptide synthetase genes) in order to produce a useful substance biosynthesized by a multi-modular biosynthetic enzyme (for example, an active polyketide or a nonribosomal peptide). The present invention provides production of a large amount of a product at an appropriate stage of the growth cycle. The useful substance biosynthesized by a multi-modular biosynthetic enzyme (for example, a polyketide or a nonribosomal peptide) produced in this manner can be used as a therapeutic agent for treating a number of diseases, depending on the type of the useful substance biosynthesized by the multi-modular biosynthetic enzyme (for example, a polyketide or a nonribosomal peptide). For example, the use of some polyketides or nonribosomal peptides produced by hosts transformed with the plasmids of the present invention as immunosuppressive drugs and anti-tumor drugs has been found, along with their use in the treatment of viral, bacterial, and parasitic infections.

More preferably, host cells for recombination production of a target useful substance biosynthesized by a multi-modular biosynthetic enzyme (for example, a polyketide or a nonribosomal peptide) can derive from any organism that can be transformed with the plasmid of the present invention. Thus, the host cells of the present invention can derive from any of prokaryotes or eukaryotes. However, preferred host cells are those constructed from actinomycetes, and more preferred host cells are hosts of the genus Streptomyces. The greatest advantages of using the hosts of the genus Streptomyces are higher production titer compared to production by heterologous expression using Escherichia coli and the existence of a posttranslational modification system essential for active expression of the type I PKS. Specific examples of the host cells of the genus Streptomyces include S. albus, S. ambofaciens, S. avermitilis, S. azureus, S. cinnamonensis, S. coelicolor, S. curacoi, S. erythraeus, S. fradiae, S. galilaeus, S. glaucescens, S. hygroscopicus, S. lividans, S. parvulus, S. peucetius, S. rimosus, S. roseofulvus, S. thermotolerans, and S. violaceoruber, and S. albus is preferable.

The host cells can be genetically engineered by deleting a naturally occurring gene encoding a multi-modular biosynthetic enzyme derived from the host cells (for example, a PKS gene or a nonribosomal peptide synthetase gene) using a standard technique (for example, homologous recombination).

A DNA encoding a multi-modular biosynthetic enzyme contained in the plasmid of the present invention (for example, a DNA encoding a PKS or nonribosomal peptide synthetase) may be a naturally occurring type, a DNA of which codon usage is changed, or a DNA of which one or two or more amino acids are changed. In one preferred embodiment, a Streptomyces PKS contains the products of three open reading frames (ORF1, ORF2, and ORF3). A PKS contains three domains: a ketosynthase (KS) domain, an acyltransferase (AT) domain, and an acyl carrier protein (ACP). A polyketide chain can be elongated by these three domains. The PKS may further contain a domain associated with modification of the main chain, such as a ketoreductase (KR) domain, a dehydratase (DH) domain, and an enoyl reductase (ER) domain. Examples of a compound prepared by the PKS include 6-deoxyerythronolide B (6-dEB), frenolicin, granaticin, tetracenomycin, 6-methylsalicylic acid, oxytetracycline, tetracycline, erythromycin, griseusin, nanaomycin, medermycin, daunorubicin, tyrosine, carbomycin, spiramycin, avermectin, monensin, nonactin, curamycin, lipomycin, rifamycin, and candicidin.

"Nonribosomal peptides" refer to a class of peptides belonging to a family of complex natural products composed of simple amino acid monomers. The nonribosomal peptides are synthesized in many bacteria or fungi by large multifunctional proteins referred to as nonribosomal peptide synthetases (NRPSs). A feature of the NRPSs is the ability to synthesize a peptide containing proteinogenic and non-proteinogenic amino acids.

The "nonribosomal peptide synthetase" (NRPS) refers to a large multifunctional protein organized in a cooperative group of active sites referred to as modules. Here, each module is required for catalyzing one cycle of peptide elongation and functional group modification. The number and order of the modules and the types of domains present in the modules on each NRPS instruct the number, order, and selection of the amino acids to be incorporated, as well as the modification associated with a specific type of elongation, thereby determining a structural variation in the obtained peptide products.

The plasmid contains a regulatory sequence operatively linked to a desired DNA encoding a multi-modular biosynthetic enzyme (for example, a DNA encoding a PKS). Expression systems suitable for use in the present invention include systems that function in eukaryotic host cells and prokaryotic host cells. However, a prokaryotic system is preferable as described above, and a system compatible with bacteria belonging to the genus Streptomyces is particularly important. The regulatory sequences to be used in such a system include a promoter, a ribosome binding site, a terminator, an enhancer, and the like. A useful promoter is a promoter that functions in host cells of the genus Streptomyces, and examples thereof include pGapdh, pErmE, pKasO, and the like, but the examples are not limited thereto.

The plasmid can also contain a selection marker. Various markers are known, including a gene that is useful in the selection of a transformed cell line and generally imparts a selectable phenotype to the transformed cells upon expression when the cells are grown in a suitable selection medium. Such markers include, for example, a gene that imparts antibiotic resistance or sensitivity to the plasmids. Alternatively, some polyketides are naturally colored, and this characteristic provides a built-in marker for selecting cells that have been successfully transformed with the construct of the present invention.

The plasmid may also contain a functional sequence functioning in the host cells. Examples of the functional sequence include a plasmid replication origin sequence, a sequence encoding an enzyme for integrating the plasmid into the host genome, a conjugation origin sequence, and the like.

The regulatory sequence, the selection marker, the functional sequence, and the like can be included in the plasmid by being integrated at suitable positions with respect to the plurality of genes encoding the multi-modular biosynthetic enzyme in the gene cluster construct.

A method of introducing the plasmid of the present invention into suitable host cells is known to those skilled in the art, and the method typically includes the use of CaCl₂ or a divalent cation and an additional agent such as DMSO. The plasmid can also be introduced into the host cells by electroporation. Once the multi-modular biosynthetic enzyme (for example, a PKS) is expressed, a colony producing the useful substance (for example, a polyketide) can be identified and isolated using a known technique.

In one preferred embodiment of the present invention, the introduction into the host cells is performed by changing the plasmid backbone by transferring a region containing the DNA encoding the multi-modular biosynthetic enzyme from the plasmid of the present invention to the plasmid of Escherichia coli (subcloning) and then transporting the plasmid from the Escherichia coli to a microorganism of the genus Streptomyces by conjugation. The DNA encoding the multi-modular biosynthetic enzyme is thus integrated into the genome of the host cells such as the microorganism of the genus Streptomyces.

In one embodiment, the plasmid of the present invention may also contain the replication origin sequences in Bacillus subtilis, Escherichia coli, and a microorganism of the genus Streptomyces, a conjugation origin sequence (OriT) for transporting the plasmid from Escherichia coli to the microorganism of the genus Streptomyces by conjugation, and a sequence encoding integrase necessary for integrating the plasmid into the genome of the microorganism of the genus Streptomyces. Such a plasmid can be collected from Bacillus subtilis, transformed into Escherichia coli without performing the subcloning for changing the plasmid backbone, and then transported from Escherichia coli to the microorganism of the genus Streptomyces by conjugation.

In a more preferred embodiment of the present invention, the plasmid contains a large DNA encoding a multi-modular biosynthetic enzyme. For example, the lipomycin biosynthetic gene cluster derived from a Streptomyces aureofaciens strain Tu117 has open reading frames corresponding to 7 modules (LipPKS1, LipPKS2, LipPKS3, LipPKS4, LipNRPS, LipMT, and LipTE).

The gene cluster construct may be any gene cluster construct long as it contains a DNA encoding a multi-modular biosynthetic enzyme (for example, a DNA encoding a domain contained in the multi-modular biosynthetic enzyme), and the type of the multi-modular biosynthetic enzyme is not particularly limited. Preferred examples of the gene cluster construct include a gene cluster constituting a PKS or NRPS. The size of the gene cluster construc is also not particularly limited. The type of the DNA encoding a multi-modular biosynthetic enzyme (for example, a DNA encoding a domain contained in the multi-modular biosynthetic enzyme) is not particularly limited, and may be any DNA having an artificially designed sequence, in addition to a DNA of a microorganism or the like having a naturally-derived sequence. In the naturally-derived sequence, one codon is usually used by the species to express the corresponding amino acid. However, in the case of heterologous expression, it is preferable to use an artificially designed sequence that is tailored to the codon usage frequency of the host. Examples of other factors that may influence the result of the heterologous expression can include the GC content (the total content of guanine and cytosine in the base sequence), repetitive sequences, and the like. The repetitive sequences lower the genetic stability, generate a risk of incorrect hybridization, and inhibit the synthesis of repetitive segments. Therefore, in the case of heterologous expression, the DNA encoding a multi-modular biosynthetic enzyme is preferably optimized in terms of the codon usage and GC content. However, it is generally difficult to optimally satisfy these conditions at the same time. For example, optimization of the codons can result in an unusually repetitive DNA sequence or high GC content.

In the present invention, the GC content of the DNA encoding a multi-modular biosynthetic enzyme is 30 to 70%. The GC content of the DNA encoding a multi-modular biosynthetic enzyme is preferably 70% or lower, 68% or lower, 65% or lower, or 60% or lower. By using the method according to the present invention, a target plasmid can be synthesized with high efficiency even when the GC content of the DNA encoding a multi-modular biosynthetic enzyme is 50% or higher, 52% or higher, 55% or higher, 58% or higher, or 60% or higher. In the present invention, it is preferable that codons are optimized in the DNA encoding a multi-modular biosynthetic enzyme so that repetition of a base sequence of 20 bp or more does not appear. It is preferable that an extreme difference in the GC contents in the DNA encoding a multi-modular biosynthetic enzyme is avoided. For example, it is preferable that the difference between the highest and lowest GC contents within a 50 bp stretch is 52% or lower. The amount of homopolymers is preferably reduced as much as possible. It is preferable that the number/length of short repetitive sequences dispersed in the DNA encoding a multi-modular biosynthetic enzyme is minimized as much as possible. Sequence repetitiveness can be evaluated using a score calculated as the total number of repeats weighted by the length of the repetitive sequence (sum (n_count x n length) for n = 5 to n = 24), as shown in Table 1. A repeat score of the DNA encoding a multi-modular biosynthetic enzyme is preferably 1000 or lower or 900 or lower. By using the present invention, the repeat score of the DNA encoding a multi-modular biosynthetic enzyme may be 600 or higher. In the case of a short repetitive sequence with a length of 5 to 10 bp, the total number of repeats is preferably 150 or less, 120 or less, 100 or less, 80 or less, or 60 or less. By using the present invention, the number of repeats of a sequence with a length of 5 to 10 bp contained in the DNA encoding a multi-modular biosynthetic enzyme may be 50 or more.

The pps gene is acquired from the plipastatin gene cluster from the NRPS gene cluster. The LipPKS_original gene is an unmodified PKS gene acquired from the lipomycin PKS gene cluster. The LipPKS_optimized gene is a codon optimized lipomycin gene used in the present invention (Tables 1 and 2).

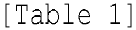

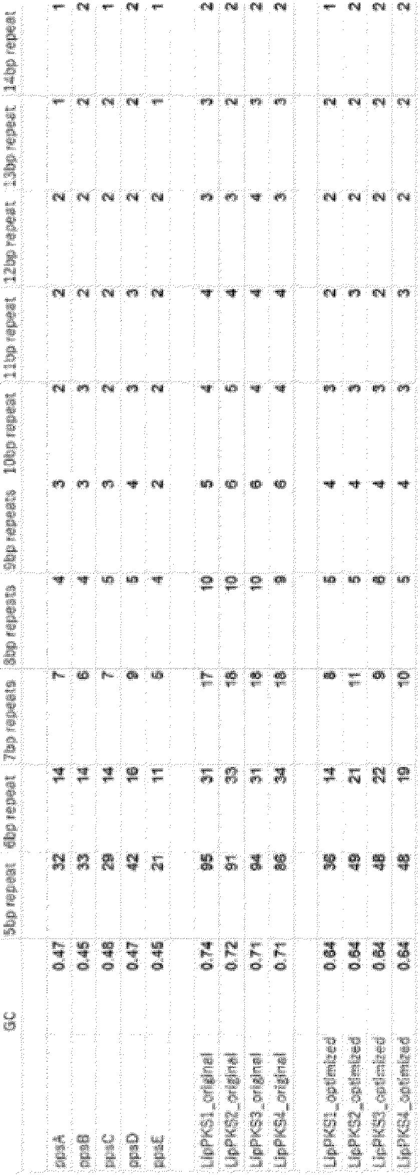

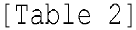

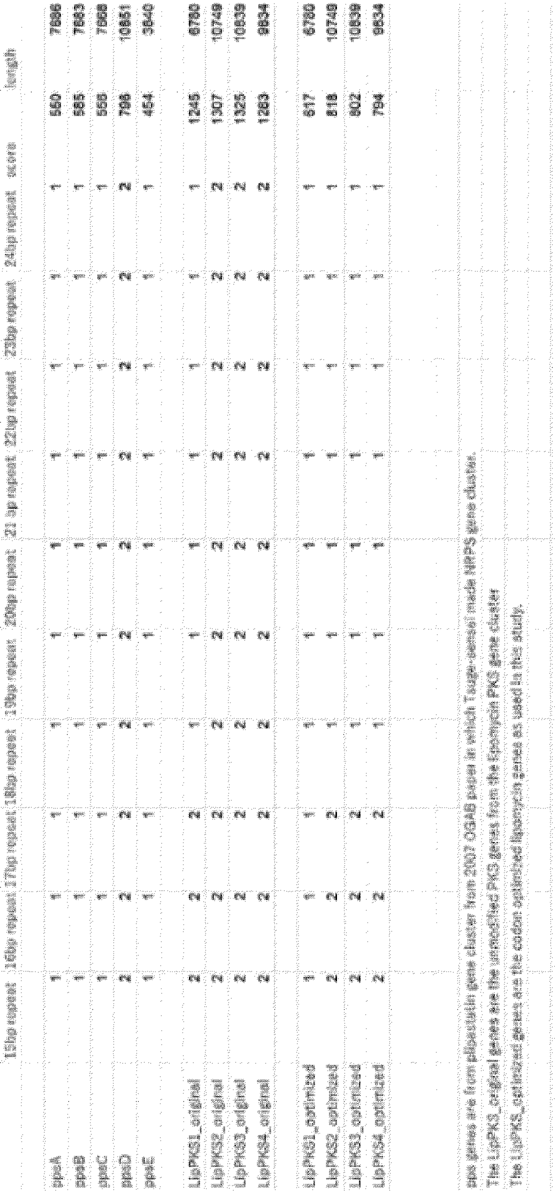

The size of the DNA encoding a multi-modular biosynthetic enzyme (for example, a DNA encoding a domain contained in the multi-modular biosynthetic enzyme) is preferably 100 kDa or more, 200 kDa or more, 300 kDa or more, 400 kDa or more, 500 kDa or more, 600 kDa or more, 700 kDa or more, 800 kDa or more, 900 kDa or more, or 1000 kDa or more.

The gene cluster construct is composed of a plurality of DNA fragments having structures that can be ligated to each other. Being able to ligate refers to the capability of the DNA fragments having adjacent sequences in the gene cluster construct to join to each other while maintaining a certain order and direction. Specific examples of the DNA fragments include fragments having the ends that can be repetitively ligated to each other while maintaining the order using the complementarity between the base sequences of the sticky ends of the fragments. There are no limitations on the structures of the sticky ends, including the difference in the forms of a 5'-end protrusion and a 3'-end protrusion, as long as it is not a batch structure (palindrome). However, it is preferable that the protruding ends can be formed during the preparation of the DNA fragments by digestion with a restriction enzyme. When an enzyme capable of recognizing a specific sequence and creating protruding ends with arbitrary sequences in the vicinity thereof is used as the restriction enzyme, the order of ligating the DNA fragments is maintained, since the sticky ends of the DNA fragments can be different from each other at each ligating site. By suitably designing the sticky end sequences of each DNA fragment, it is possible to obtain a gene cluster construct containing a plurality of DNAs encoding a multi-modular biosynthetic enzyme (for example, DNAs encoding a PKS or an NRPS) in which each DNA fragment is aligned in a predetermined order. Examples of the restriction enzyme include, in addition to the restriction enzymes generally used in the molecular biology, artificial restriction enzymes such as TALEN and ZNF and CRISPR technique-related enzymes that can generate sticky ends such as CRISPR-Cpf1. A Type II restriction enzyme is preferably used, such as AarI, AlwNI, BbsI, BbvI, BcoDI, BfuAI, BglI, BsaI, BsaXI, BsmAI, BsmBI, BsmFI, BspMI, BspQI, BtgZI, DraIII, FokI, PflMI, SfaNI, and SfiI. Optimal sticky ends can be determined using NEBeta^{™} Tools. The number of bases in the sticky end is preferably 3 to 6 and more preferably 3 or 4. The number of bases contained in one DNA fragment is preferably 1 to 5 kb. The number of bases in one DNA fragment may be 2 kb or more, 3 kb or more, or 4 kb or more. Regarding the number of types of the restriction enzymes used for generating the sticky ends, it is preferable that cleavage is performed by a single type of restriction enzyme in the excision of one DNA fragment. Although it is not necessary that all DNA fragments are obtained by the digestion with the same type of restriction enzyme, it is favorable that the total number of the types of the restriction enzymes used is small. The total number of the types of the restriction enzymes used is preferably three types or less, more preferably two types or less, and even more preferably one type.

The gene cluster construct may include other genes in addition to the DNA encoding a multi-modular biosynthetic enzyme (for example, a DNA encoding a domain contained in the multi-modular biosynthetic enzyme). For example, in a case where the gene cluster construct includes the PKS gene, it is important that the gene cluster construct includes other non-PKS genes in addition to the PKS gene, for modification of a final polyketide product, extracellular transport of the polyketide, or impartment of resistance to an antibiotic polyketide.

The number of types of the DNA fragments is 3 to 60 (types), preferably 5 to 50 (types), more preferably 8 to 40 (types), and even more preferably 10 to 30 (types).

In the step (A) in the preparation method for the gene cluster construct of the present invention, a plurality of DNA fragments are prepared, which are capable of reconstructing the gene cluster construct and have structures that can be ligated to each other. Sticky ends having structures that can be ligated to each other may be formed in each of the DNA fragments by the cleavage with the restriction enzyme. Alternatively, each of the DNA fragments having sticky ends having structures that can be ligated to each other may be prepared by synthesizing a plasmid containing each DNA fragment and cleaving the plasmid with a restriction enzyme. The GC content of each DNA fragment may be 65% or lower. It is preferable that repetition of a base sequence of 20 bp or more does not appear in each DNA fragment. The lengths of the DNA fragments are not necessarily the same as each other. The lengths of the DNA fragments having the same sticky sequences are preferably the same.

In the step (B) in the preparation method for the gene cluster construct of the present invention, the plurality of DNA fragments prepared in the step (A) are mixed in a solution. The mixing is preferably performed so that the ratios between the molar concentrations of the DNA fragments are all 0.8 to 1.2, preferably 0.9 to 1.1, more preferably 0.95 to 1.05, and even more preferably about 1.0. The mixing of the DNA fragments is performed in the presence of a plasmid for accumulation as necessary to accumulate the DNA fragments, whereby a construct including a tandem repeat of a cluster of a plurality of genes encoding a multi-modular biosynthetic enzyme is obtained. By introducing the obtained gene cluster construct into Bacillus subtilis, a library of plasmids containing the gene cluster encoding a multi-modular biosynthetic enzyme is obtained. The multi-modular biosynthetic enzyme can be expressed in host cells by transferring the gene cluster of the multi-modular biosynthetic enzyme in the plasmid library to a shuttle plasmid and transporting the gene cluster to suitable host cells such as bacteria of the genus Streptomyces by conjugation with Escherichia coli. For example, in a case where the combinatorial library is constructed from plasmids having promoters with different strength or expression levels for each gene, a preferable combination of promoters can be found for high expression by expressing the multi-modular biosynthetic enzyme in suitable host cells.

In the step (P) in the preparation method for the gene cluster construct of the present invention, a plurality of plasmids (seed plasmids) are prepared. Taking the steps (A1) and (B1) into consideration, the seed plasmids may have structures in which a suitable restriction enzyme recognition sequence is inserted at the end or in the vicinity of each of the DNA fragments in accordance with the designing thereof, so that the seed plasmids can be divided into the DNA fragments after building of the accumulated product. As the restriction enzyme, an enzyme capable of creating sticky ends having arbitrary sequences is preferably used, such as AarI, AlwNI, BbsI, BbvI, BcoDI, BfuAI, BglI, BsaI, BsaXI, BsmAI, BsmBI, BsmFI, BspMI, BspQI, BtgZI, DraIIIFokI, PflMI, SfaNI, SfiI, or the like. A plurality of sticky sequences obtained by treatment with these restriction enzymes may be the only sequences within a single seed plasmid. In addition, a seed plasmid group may have the same sticky sequences on the same chain in the same order in a recombination unit of the combinatorial library (although one DNA fragment coincides with the unit in many cases, the recombination unit may consist of a plurality of DNA fragments in some seed plasmids).

In the step (A1) in the preparation method for the gene cluster construct of the present invention, a mixture solution of the plurality of types of DNA fragments is prepared by treating the plurality of types of seed plasmids with restriction enzymes suitable to each of the plasmids. Examples of the restriction enzymes are the same as those described above. The obtained product of the restriction enzyme digestion contains desired DNA fragments and fragments derived from the plasmids. In one preferred embodiment of the present invention, since the sizes of the DNA fragments used in the present invention and the fragments derived from the seed plasmids are different, a nearly equimolar mixture of the desired DNA fragments is obtained by subjecting the DNA fragments and the fragments derived from the seed plasmids to size fractionation by electrophoresis. A nearly equimolar mixture refers to a mixture in which the ratios between the molar concentrations of the DNA fragments are all in the range of 0.8 to 1.2, preferably in the range of 0.9 to 1.1, more preferably in the range of 0.95 to 1.05, and even more preferably about 1.0.

In the step (B1) in the preparation method for the gene cluster construct of the present invention, the nearly equimolar mixture of the desired DNA fragments obtained in the step (A1) is reaccumulated in the presence of a plasmid for accumulation as necessary, whereby a gene cluster construct including a tandem repeat of a cluster of a plurality of genes encoding a multi-modular biosynthetic enzyme is obtained. A plasmid library is obtained in the same manner as in (B) described above using the obtained gene cluster construct. A highly-expressed multi-modular biosynthetic enzyme gene cluster can be sorted by expressing the multi-modular biosynthetic enzyme gene in the host cells using the plasmid library in which the gene sequences constructing the cluster or at least one of the regulatory sequences such as the promoter strength in the host cells is variously changed.

Although it is possible to prepare the gene cluster construct by subjecting the DNA fragment mixture solution to ligation using a DNA ligase or the like, starting materials for the gene accumulation are not limited only to each DNA fragment obtained in the step (P), and an accumulated product prepared by any accumulation method may be used as long as it is a structure that can be finally divided into each DNA fragment as described above. Here, the ratios between the molar concentrations of the DNA fragments in the DNA fragment mixture solution are all in the range of 0.8 to 1.2, preferably in the range of 0.9 to 1.1, more preferably in the range of 0.95 to 1.05, and even more preferably about 1.0.

Although a DNA fragment ligation method is not particularly limited, the ligation is preferably performed in the presence of polyethylene glycol and a salt. The salt is preferably a monovalent alkali metal salt. It is preferable that the ligation is performed using T4 DNA polymerase at 37°C for 30 minutes or longer.

For example, in a case where a DNA encoding a PKS is represented by P-Q-R-S in which four domains of P, Q, R, and S are ligated in this order, the gene cluster construct includes a tandem repeat represented by -(P-Q-R-S)ₙ- (n is an integer of 2 or greater). It is preferable that a regulatory sequence such as a promoter functioning in the host cells, a ribosome binding sequence (RBS sequence), and an enhancer is added to the 5' end or 3' end of each ORF (P-Q-R-S) in the gene cluster construct. Furthermore, a sequence such as a functional sequence functioning in the host cells and a selection marker may be added to the 5' end or 3' end of each ORF (P-Q-R-S) in the gene cluster construct. In addition, the gene cluster construct preferably contains a replication origin effective in Bacillus subtilis. Examples of the replication origin in the case of Bacillus subtilis include those having the theta replication mechanism, specifically, the sequence of the replication origin contained in the plasmid of pTB19 (Imanaka, T., et al., J. Gen. Microbioi., 130, 1399-1408. (1984)), pLS32 (Tanaka, T and Ogra, M., FEES Lett., 422, 243-246. (1998), pAMβ1 (Swinfield, T. J., et al., Gene, 87, 79-90. (1990)), or the like.

By culturing competent Bacillus subtilis cells along with the gene cluster construct, the gene cluster construct is taken up by the Bacillus subtilis, and the plasmid containing the DNA encoding a multi-modular biosynthetic enzyme (for example, a DNA encoding a PKS or an NRPS) is formed. As a method of obtaining the competent Bacillus subtilis, the method described in Anagnostopoulou, C. and Spizizen, J., J. Bacteriol., 81, 741-746 (1961) is preferably used. The fluid volume of a gene cluster construct solution added to the competent Bacillus subtilis cells is not particularly limited. The fluid volume of the gene cluster construct solution is preferably in a range of 1/20 of the fluid volume of a culture solution of the competent Bacillus subtilis cells to the same volume as the fluid volume of the culture solution, and more preferably half the fluid volume of the culture solution.

Regarding the tandem repeat of the DNA encoding a multi-modular biosynthetic enzyme (for example, a DNA encoding a PKS or an NRPS), at least one tandem repeat, preferably one DNA encoding a multi-modular biosynthetic enzyme (for example, a DNA encoding a PKS or an NRPS), is integrated into the plasmid during the formation of the plasmid. As a method of purifying the plasmid from Bacillus subtilis, a known method can be used.

Whether or not the plasmids obtained by the method described above have the target DNAs encoding a multi-modular biosynthetic enzyme (for example, DNAs encoding a PKS or an NRPS) can be confirmed by size patterns of the fragments generated by the restriction enzyme cleavage, a PCR method, or a sequencing method.

The terminator is not particularly limited as long as it functions in the host cells, and examples thereof include a terminator derived from fd phage (fd-ter), a terminator derived from T4 phage (T4-ter), a terminator derived from T7 phage (T7-ter), and the like. Among these, a terminator derived from fd phage is particularly preferable, since it has a rather large stabilizing effect described above.

As for the ribosome binding sequence (RBS), a known RBS can be used.

In one embodiment of the present invention, a transformant obtained by introducing the plasmid containing the DNA encoding a multi-modular biosynthetic enzyme (for example, a DNA encoding a PKS or an NRPS) into the host cells is cultured, and a useful substance (for example, a polyketide or a nonribosomal peptide) can be obtained from the culture. The "culture" refers to any of the culture supernatant, cultured cells, cultured bacterial cells, or a homogenate of the cells or bacterial cells. A method of culturing the transformant of the present invention can be performed in accordance with an ordinary method used for host culturing.

Any of a natural medium or a synthetic medium may be used as a medium for culturing the transformant of the present invention, as long as the medium contains a carbon source, a nitrogen source, inorganic salts, and the like that the host can assimilate, whereby the culturing of the transformant can be performed efficiently. Examples of the carbon source include a carbohydrate such as glucose, galactose, fructose, sucrose, raffinose, and starch, an organic acid such as acetic acid and propionic acid, and alcohols such as ethanol and propanol. Examples of the nitrogen source include ammonia, an ammonium salt of an inorganic acid or an organic acid, such as ammonium chloride, ammonium sulfate, ammonium acetate, and ammonium phosphate, and other nitrogen-containing compounds. In addition, peptone, meat extract, corn steep liquor, various amino acids, or the like may be used. Examples of the inorganic substance include monopotassium phosphate, dipotassium phosphate, magnesium phosphate, magnesium sulfate, sodium chloride, ferrous sulfate, manganese sulfate, copper sulfate, and calcium carbonate.

The culturing is generally performed under an aerobic condition such as shaking culture or aeration and agitation culture at 28 to 38°C. pH adjustment is performed using an inorganic or organic acid, an alkaline solution, or the like.

When the culturing is performed under the above culture conditions, the useful substance (for example, a polyketide) can be produced at a high yield.

In a case where the useful substance (for example, a polyketide) is produced within the bacterial cells or the cells, the useful substance can be collected after the culturing by performing homogenization treatment or the like to homogenize the bacterial cells or the cells. On the other hand, in a case where the useful substance (for example, a polyketide) is secreted outside the bacterial cells or the cells, the culture solution is used as it is, or the bacterial cells or the cells are removed by centrifugation or the like. Next, the useful substance is collected from the culture by extraction by ammonium sulfate precipitation or the like, and further subjected to isolation and purification as necessary using various chromatography techniques or the like.

A modular PKS sequentially catalyzes a plurality of chemical reactions within the modules, and a spatial arrangement of each domain is important. In the past few years, some studies have identified optimal fusion boundary candidates for PKS modules and domain swapping. However, the spatial arrangement can vary from module to module, since protein sequences are different from each other. This implies that the optimal boundary positions in combinatorial biosynthesis can rely on the contents. Therefore, by using the present invention, targeted changes such as AT domain swapping can be performed using various domain boundaries, and the activity of the obtained enzyme can be evaluated in vitro.

### Examples

Hereinafter, the present invention will be described based on Examples, but the present invention is not limited to these Examples.

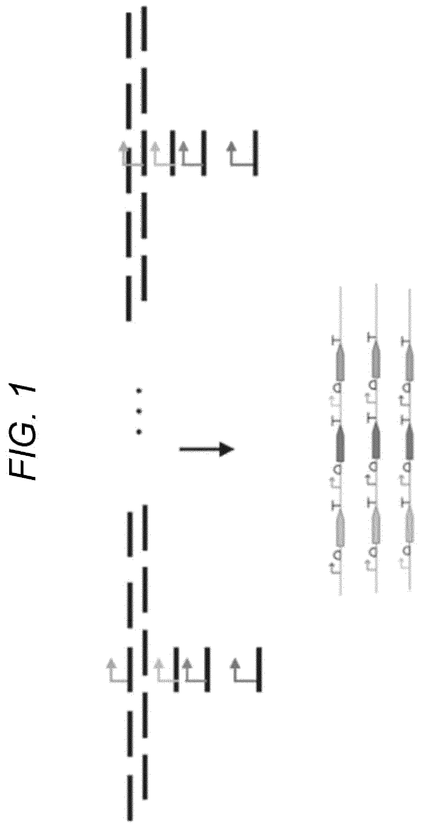

Fig. 1 is a schematic diagram illustrating the procedures in Examples 1 and 2. In Fig. 1, the schematic diagrams below the arrow each represent a gene cluster construct including a plurality of genes encoding a multi-modular biosynthetic enzyme. In each gene cluster construct, a plurality of combinations of promoters, RBSs, genes encoding a multi-modular biosynthetic enzyme (CDSs), and terminators are aligned with spacer sequences interposed therebetween. In Examples 1 and 2, the gene cluster constructs were obtained by ligating a plurality of DNA fragments having structures that can be ligated to each other (in Fig. 1, the schematic diagrams above the arrow). In Example 1, all DNA fragments were ligated at once to obtain the gene cluster construct. In Example 2, the DNA fragments were divided into three groups, and a gene cluster construct was first obtained as plasmids using each group. Then, after combining the plasmids obtained using the three groups, the plasmids were disintegrated into the DNA fragments again, and the DNA fragments were ligated and inherited to obtain the cluster construct. The ligation of the DNA fragments also involves ligation of DNA fragments serving as vector backbones.

### Examples 1 and 2

### <Designing of DNA>

The following gene sequences were used as the plurality of genes encoding a multi-modular biosynthetic enzyme. The following gene sequences were selected from the lipomycin biosynthetic gene cluster of the Streptomyces aureofaciens strain Tu117.
LipPKS1
LipPKS2
LipPKS3
LipPKS4
LipNRPS
LipMT
LipTE

All CDSs were custom designed and codon optimized so that the GC contents were lower than 70%, and repeats of 20 bp or more did not exist. Balance between the requirements for efficient expression in the target production host (Streptomyces albus) is also achieved.
GC contents before codon optimization (%):
   LipPKS1: 74.2%
   LipPKS2: 72.3%
   LipPKS3: 71.3%
   LipPKS4: 71.3%
   LipNRPS: 75.2%
   LipMT: 71.1%
   LipTE: 74.5%
GC contents after codon optimization (%):
   LipPKS1: 63.7%
   LipPKS2: 63.7%
   LipPKS3: 63.9%
   LipPKS4: 63.9%
   LipNRPS: 65.3%
   LipMT: 59.6%
   LipTE: 65.7%

All native restriction enzyme recognition sites used in assembly of the combinatorial library and vector transport were deleted from the CDSs (BsmbI, BsaI, and AarI) .

The obtained sequence-optimized CDSs were used for designing a new gene cluster using the promoter and RBS sequence of Streptomyces albus.

Specifically,
the promoter and RBS were positioned before each PKS CDS, and the terminator and the spacer sequence were positioned at the end of each CDS. The promoter sequences used in Examples are indicated by SEQ ID NOs: 14 to 23.

The LipNRPS, LipMT, and LipTE genes were organized into a single operon under the regulation of one promoter. Each of the three CDSs was given a unique RBS. The terminator was positioned at the end of the operon.

Thus, the designed gene cluster construct is composed of repeats of a sequence containing the vector backbone sequence and the LipPKS1 gene (gene 1), the LipPKS2 gene (gene 2), the LipPKS3 gene (gene 3), LipPKS4 (gene 4), and the above-described operon (gene 5) in this order, each gene containing the promoter and RBS before the CDS and the terminator and the spacer sequence after the

### CDS.

### <Assembly>

### Example 1 (Direct OGAB Method)

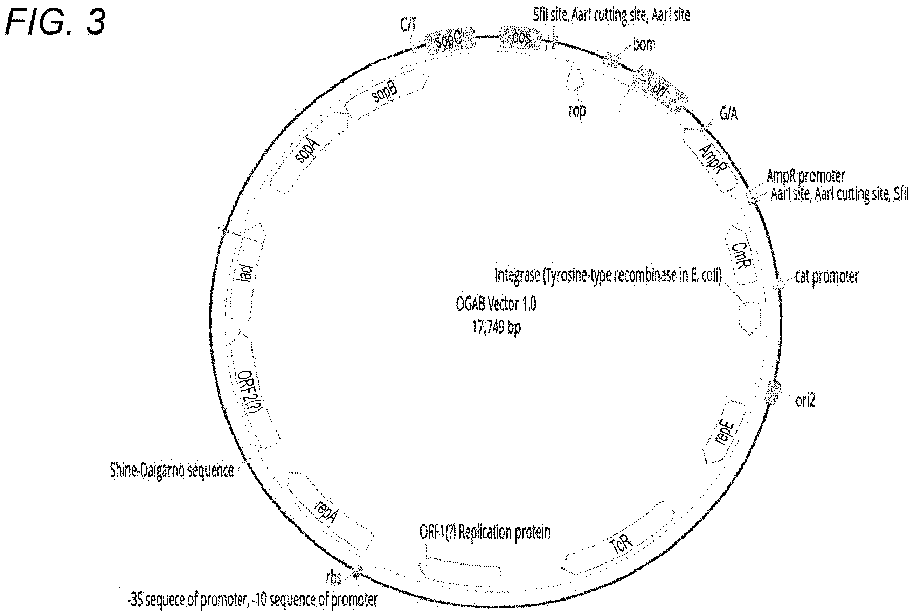

The concentrations of all DNA fragments (SEQ ID NOs: 1 to 13 and 28 to 37) containing promoter variants were measured using a UV spectrophotometer (Thermofisher Nanodrop) and adjusted so that an equimolar fragment mixture was created. Treatment with a DNase (Lucigen Corporation) was performed to remove contaminating linear DNAs. The concentration was normalized to 100 ng/ul for each DNA fragment. 500 ng of each DNA fragment was collected in a tube and treated with BsaI-HFv2 restriction enzyme (NEB). In order to purify the DNAs digested by the restriction enzyme, phenol-chloroform treatment, butanol treatment, and ethanol precipitation were performed. Target fragments were removed from the digested plasmid mixture by performing gel extraction using dialysis tubing, and the obtained digested target fragments were purified by ethanol precipitation. The digested fragments were mixed with a digested vector (OGAB Vector 1.0), 1 µl of T4 DNA Ligase (Takara Bio Inc.), and a ligation buffer, and ligation was completed by incubating the mixture at 37°C for 3 hours, whereby the designed gene cluster construct was obtained. A DNA ligation solution containing the gene cluster construct was mixed with competent Bacillus subtilis cells, and the cells were spread on a tetracycline selection plate after a short period of incubation at 37°C. After the period of colony growth, the transformants were picked from the plate and grown overnight in 2 ml of LB at 37°C. Plasmid extraction was performed as in the conventional protocol, and whether the obtained plasmids were assembled as expected was confirmed by a restriction digestion pattern (pattern of cleavage by restriction enzyme: NotI). The base sequence of OGAB Vector 1.0 is as shown in SEQ ID NO: 24. Fig. 3 is a vector map of OGAB Vector 1.0.

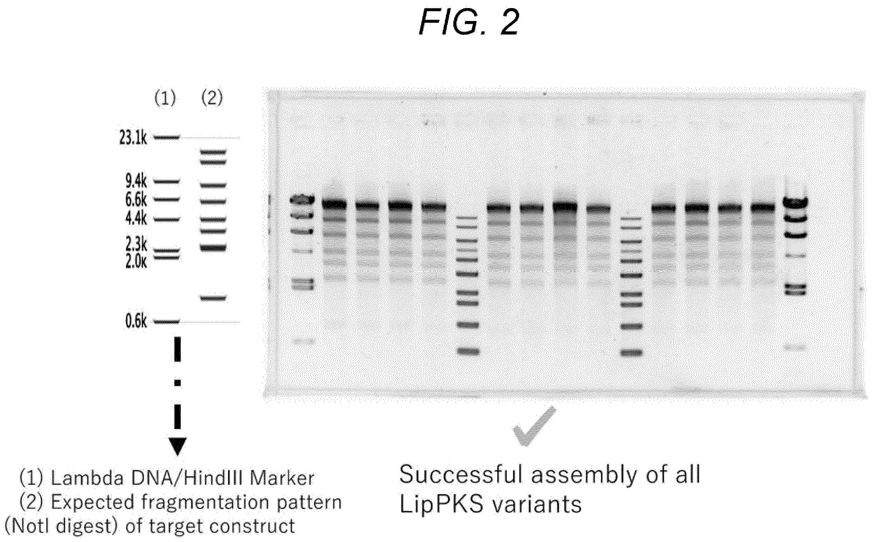

Fig. 2 is a photograph showing an example of restriction digestion patterns of the obtained plasmids. Among 24 colonies that were analyzed, 20 colonies were assembled as expected (20/24 = 83%).

### Example 2 (Standard OGAB Method)

Group 1: DNA fragments (SEQ ID NOs: 1 to 13)
Group 2: DNA fragments (SEQ ID NOs: 1 to 3, 5 to 9, and 28 to 32)
Group 3: DNA fragments (SEQ ID NOs: 1 to 3, 5 to 9, and 33 to 37)

First, plasmids were obtained for each of the above Groups 1 to 3 by the same procedures as in Example 1. Next, reorganized plasmids were obtained by the same procedures as in Example 1 using the plasmids of Groups 1 to 3 instead of the DNA fragments. As in Example 1, whether the obtained plasmids were assembled as expected was confirmed by restriction digestion patterns. As a result, among 24 colonies that were analyzed, 23 colonies were assembled as expected (23/24 = 96%).

In both Examples 1 and 2, the types of promoters integrated into five promoter positions (refer to Fig. 1) were random, and no significant bias was observed. That is, an unbiased combinatorial library was constructed. Furthermore, the base sequences of the plasmids that were confirmed to be assembled as expected by the restriction digestion patterns were determined using Flongle (manufactured by Oxford Nanopore Technologies plc), and as a result, the base sequences of the plasmids matched well with the designed base sequence (99.96% match within about 60 kbp) .

### Example 3

The LipPKS gene cluster was excised from the plasmid obtained using only the Group 1 DNA fragments of Example 2 by the restriction enzyme AarI, ligated to the restriction enzyme AarI site of an E. coli-Streptomyces shuttle vector (pSYN0002, SEQ ID NO: 38), and cloned in E. coli (ET12567 (pUZ8002)). Using this E. coli, the LipPKS gene cluster was transferred to Streptomyces by conjugation, and the expression of the LipPKS gene cluster was confirmed.

### Example 4

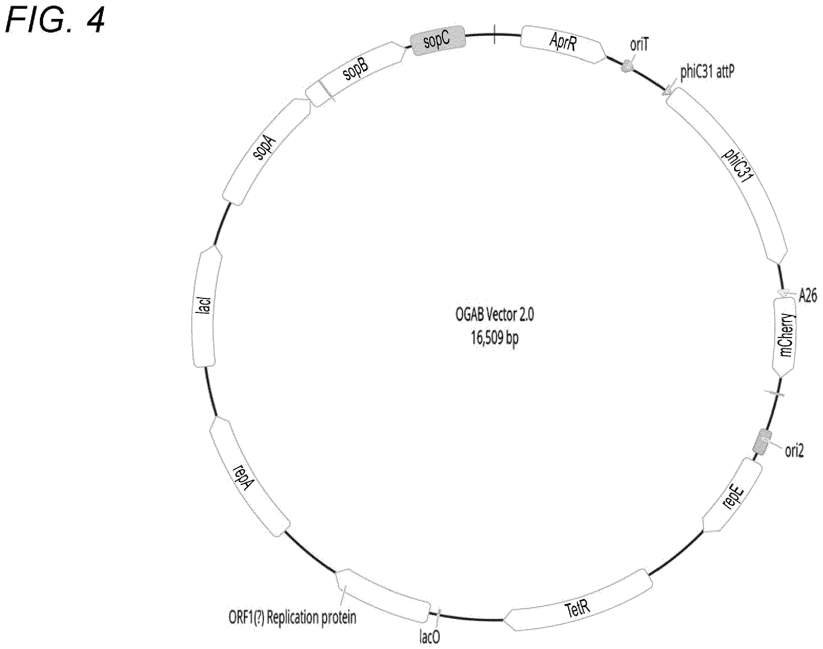

Plasmids were obtained in the same manner as in Example 1, except that a direct shuttle vector (OGAB Vector 2.0, SEQ ID NO: 25) was used instead of OGAB Vector 1.0 of Example 1, and the DNA fragments having the base sequences of SEQ ID NOs: 1 to 13 were used as the DNA fragments. Fig. 4 is a vector map of OGAB Vector 2.0. OGAB Vector 2.0 contains the replication origin sequences in Bacillus subtilis, Escherichia coli, and a microorganism of the genus Streptomyces, a conjugation origin sequence (OriT) for transporting the plasmids from Escherichia coli to the microorganism of the genus Streptomyces by conjugation, and a site-specific recombination system necessary for integration into the genome of the microorganism of the genus Streptomyces (the sequence encoding phiC31 integrase and the phiC31 attP sequence). The obtained plasmids were transformed into E. coli, the LipPKS gene cluster was transferred to Streptomyces by conjugation using this E. coli, and the expression of the LipPKS gene cluster was confirmed.

### Example 5

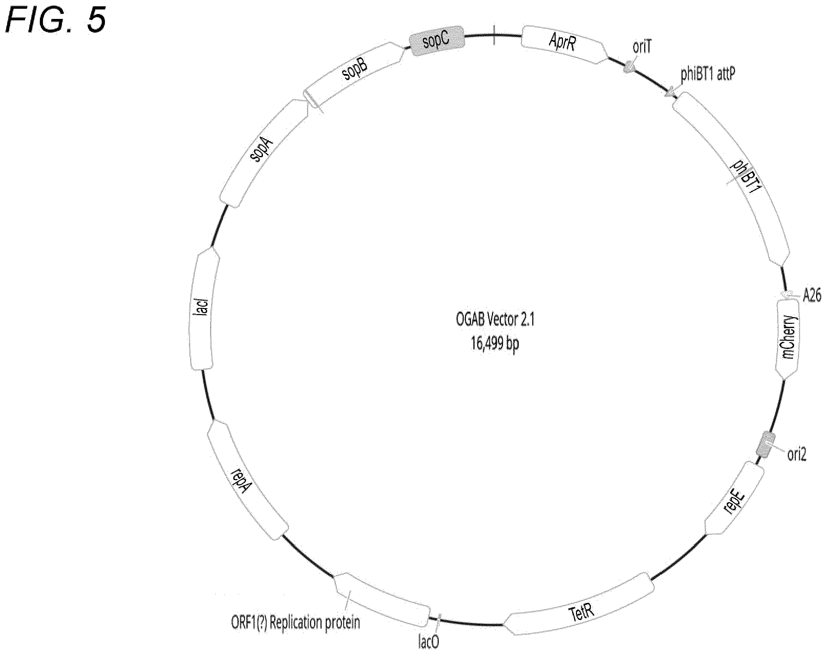

The LipPKS gene cluster was transferred to Streptomyces by conjugation, and the expression of the LipPKS gene cluster was confirmed by the same procedures as those in Example 4, except that a direct shuttle vector (OGAB Vector 2.1, SEQ ID NO: 26) was used instead of OGAB Vector 2.0 of Example 4. Fig. 5 is a vector map of OGAB Vector 2.1. OGAB Vector 2.1 is obtained by changing the site-specific recombination system in OGAB Vector 2.0, which is necessary for integration into the genome of the microorganism of the genus Streptomyces, to the sequence encoding phiBT1 integrase and the phiBT1 attP sequence.

### Example 6

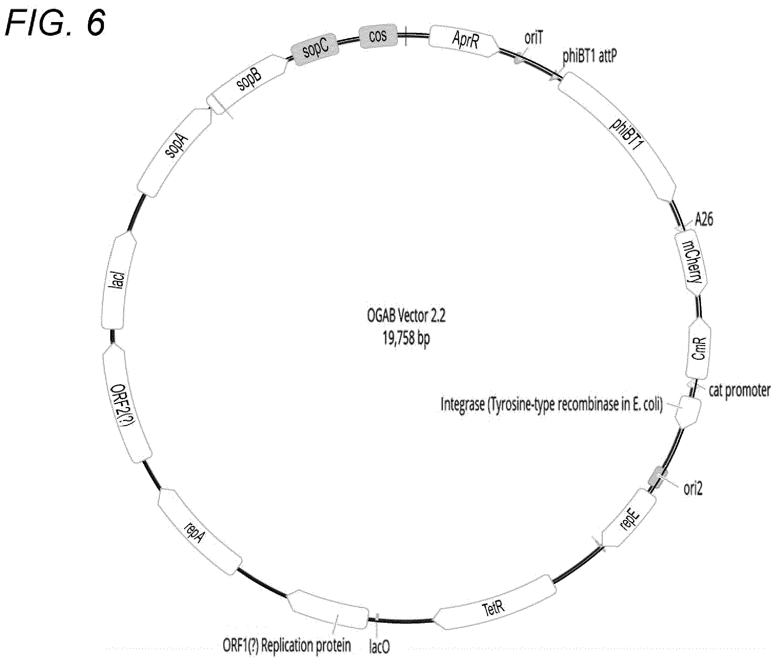

The LipPKS gene cluster was transferred to Streptomyces by conjugation, and the expression of the LipPKS gene cluster was confirmed by the same procedures as those in Example 4, except that a direct shuttle vector (OGAB Vector 2.2, SEQ ID NO: 27) was used instead of OGAB Vector 2.0 of Example 4. Fig. 6 is a vector map of OGAB Vector 2.2. OGAB Vector 2.2 is obtained by adding sequences encoding an integrase (Tyrosine-type recombinase) and a chloramphenicol-resistance gene to OGAB Vector 2.1.

## Claims

1. A method of preparing a gene cluster construct including a plurality of genes encoding a multi-modular biosynthetic enzyme, the method comprising:
(A) a step of preparing a plurality of DNA fragments which are capable of reconstructing the gene cluster construct and have structures that can be ligated to each other; and
(B) a step of ligating the plurality of DNA fragments prepared in the step (A) to each other by mixing the plurality of DNA fragments in a solution to obtain the gene cluster construct.

2. The method according to claim 1, wherein the DNA fragments are each contained in plasmids, and the method further includes a step of preparing the DNA fragments of the step (A) from the plasmids.

3. A method of preparing a gene cluster construct including a plurality of genes encoding a multi-modular biosynthetic enzyme, the method comprising the following steps:
(P) a step of preparing a plurality of types of plasmids containing a plurality of DNA fragments having structures that can be ligated to each other;
(A1) a step of preparing a mixture solution of the plurality of types of DNA fragments by treating the plurality of types of plasmids with restriction enzymes suitable to each of the plasmids; and
(B1) a step of reaccumulating the DNA fragments using the mixture solution of the DNA fragments obtained in the step (A1) to obtain the gene cluster construct.

4. The method according to claim 3, wherein, in the step (P), the plurality of types of plasmids containing the plurality of DNA fragments having the structures that can be ligated to each other are prepared by using Bacillus subtilis.

5. The method according to any one of claims 1 to 4, wherein the multi-modular biosynthetic enzyme is a modular polyketide synthase (PKS).

6. The method according to claim 3, wherein the restriction enzymes are Type II restriction enzymes.

7. A preparation method for a combinatorial library of plasmids comprising: transforming the gene cluster construct prepared by the method according to any one of claims 1 to 6 into Bacillus subtilis; and recovering plasmid DNAs from the Bacillus subtilis.

8. The method according to claim 3, wherein the step (A1) and the step (B1) are performed by selecting a plurality of types of the plasmids obtained by the preparation method according to claim 7 and reusing the selected plasmids as the plasmids in the step (A1).

9. A combinatorial library of plasmids obtained by the preparation method according to claim 7.

10. A method of producing a multi-modular biosynthetic enzyme comprising: transforming the plasmids according to claim 9 into host cells.

11. The method according to claim 10, wherein the host cells are microorganisms belonging to the genus Streptomyces.
